Europäisches Patentamt

European Patent Office

Office européen des brevets

(19)

(11) **EP 1 554 982 A1**

(12) **EUROPEAN PATENT APPLICATION**
published in accordance with Art. 158(3) EPC

(43) Date of publication:
20.07.2005 Bulletin 2005/29

(51) Int Cl.7: **A61B 8/08**

(21) Application number: 03756668.4

(22) Date of filing: 17.10.2003

(86) International application number:
**PCT/JP2003/013301**

(87) International publication number:
**WO 2004/039262 (13.05.2004 Gazette 2004/20)**

(84) Designated Contracting States:
**AT BE BG CH CY CZ DE DK EE ES FI FR GB GR HU IE IT LI LU MC NL PT RO SE SI SK TR**

(30) Priority: 18.10.2002 JP 2002304399

(71) Applicant: Hitachi Medical Corporation
Tokyo 100-0047 (JP)

(72) Inventors:
• **MATSUMURA, Takeshi**
Kashiwa-shi, Chiba 277-0825 (JP)

• **TAMANO, Satoshi**
Kashiwa-shi, Chiba 277-0054 (JP)
• **MITAKE, Tsuyoshi**
Noda-shi, Chiba 278-0022 (JP)
• **SIINA, Tsuyoshi**
Tsukuba-shi, Ibaraki 305-0032 (JP)
• **YAMAKAWA, Makoto**
Tsukuba-shi, Ibaraki 305-0031 (JP)

(74) Representative: Strehl Schübel-Hopf & Partner
Maximilianstrasse 54
80538 München (DE)

(54) **ULTRASONOGRAPHIC DEVICE**

(57) An ultrasound diagnostic apparatus according to the present invention includes means for generating a tomographic image from a reflected echo signal received by the probe corresponding to an ultrasound wave transmitted by a probe put on an object to be examined, means for measuring a displacement of the object's tissue and calculating elasticity information on the basis of a reflected echo signal received from the object corresponding to an ultrasound wave transmitted while the pressure applied when the probe is put on the object is changed, and generating a color elasticity image from the elasticity information, means for generating a translucent image from the color elasticity image on the basis of image information of either the tomographic image or the color elasticity image, and overlapping the translucent image and the tomographic image, and means for displaying the combined image. With this structure, relation between the tissue and its elastic behavior can be properly diagnosed with the ultrasound diagnostic image.

Fig.1

**Description**

Technical Field

The present invention relates to a technique of forming a combined image using tomographic image information of an object to be examined and elasticity image information presenting stiffness and softness and the like of the object's tissue and displaying the combined image as a translucent image.

Background of the Invention

In recent years, in the field of ultrasound diagnostic apparatus it is proposed to form a combined image using tomographic image information of an object to be examined and elasticity image information presenting stiffness and softness of the object's tissue.

An example thereof is mentioned in Japanese Unexamined Patent Publication No.2000-60853 (Patent document 1). That is, the technique is designed to measure a displacement of the object's tissue on the basis of a time-sequential reflected echo signal generated from the object, calculate the elasticity information such as stiffness, softness, distortion, an elasticity ratio of the tissue, and the like from the measured displacement, form a color elasticity image from the calculated elasticity information, and judge a stiff region or a soft region of the object's tissue by superposing the color elasticity image on an independently formed tomographic image and parallelizing them.

On the other hand, Japanese Unexamined Patent Publication No.Hei.8-173428 (Patent document 2) discloses a technique of turning a color Doppler image showing bloodstream information in color into a translucent image and superposing it on a tomographic image in the display on the basis of their positional relation.

Summary of the Invention

An ultrasound diagnostic apparatus according to the present invention includes a probe put onto the object for transmitting and receiving an ultrasound wave, means for generating a tomographic image from a reflected echo signal received by the probe from the object corresponding to the transmitted ultrasound wave, means for measuring the displacement of the object's tissue and calculating elasticity information on the basis of received reflected echo signal corresponding to an ultrasound wave transmitted from the probe while pressure applied by the probe to the object is varied, and generating a color elasticity image from the elasticity information, means for overlapping the tomographic image and the color elasticity image into a translucent image on the basis of image information of at least either the tomographic image or the color elasticity image, and means for selectively displaying the tomographic image, the color elasticity image, and the translucent combined image.

With this structure, in the ultrasound diagnostic image the relation between the tissue and its elastic behavior can be properly diagnosed.

According to one desired embodiment of the present invention, the translucent image generating means includes means for overlapping the tomographic image data and the color elasticity image data into a translucent image data on the basis of combination ratios of the tomographic image data generated by the tomographic image generating means and of the color elasticity image data generated by the elasticity image generating means.

Further, according to a desirable embodiment of the present invention, the combination ratios of the tomographic image data and of the color elasticity image data are determined on the basis of the elasticity information calculated by the elasticity image generating unit. Further, pressure measuring unit for measuring pressure information applied to the object by the probe is attached to the probe, and the combination ratios of the tomographic image data and of the color elasticity image data are determined on the basis of the pressure information measured by the pressure measuring unit. Further, the apparatus further includes means for calculating a mean value of the tomographic image, means for calculating a mean value of color brightness of the elasticity image, means for calculating a coefficient with which thus calculated mean values are equalized, and means for calculating a combination ratio of the elasticity image by multiplying each image pixel by the calculated coefficient.

Further, according to a desired embodiment of the present invention, the apparatus further includes means for variably setting a region of interest (ROI) of the color elasticity image data, and translucent image data is combined in the ROI.

With this structure, a region to be calculated can be reduced by, e.g. making small a displayed region of the elasticity image, whereby the frame rate is improved in the measurement of the tomographic image and the elasticity image.

Further, according to a desirable embodiment of the present invention, the translucent image generating means further includes calculating means for separating brightness information of the tomographic image into three primary colors and calculating a translucent image information combined from thus separated three primary colors and hue information of the color elasticity image.

Further, according to a desirable embodiment of the present invention, the display means further includes means for selectively displaying a numerical value of the combination ratio of either the elasticity image or the tomographic image on the display unit. The numerical value displayed by this display setting means can be arbitrarily changed, and the display control means displays the changed numerical value in response to the change.

With this structure, the combination ratios of the tomographic image and of the elasticity image is apparently shown, and the operator can arbitrarily set the combination ratios.

Further, according to a desirable embodiment of the present invention, the elasticity image generating unit includes outline detecting means for detecting a borderline between tissues having different elastic behavior in the color elasticity image on the basis of a predetermined threshold of the elasticity information and means for generating a combined image of the detected borderline and the generated tomographic image.

Because the outline image of the elasticity image is superposed on the tomographic image in the display, shape information obtained in the tomographic image is emphasized in the display and their positional relation becomes more apparent.

Further, according to a desirable embodiment of the present invention, the display selecting means includes means for storing the tomographic image data and the elasticity image data, and switches the stored tomographic image data and elasticity image data to selectively display them on the display unit.

With this structure, the tomographic image and the elasticity image can be parallelized due to an afterimage effect of the observer's eyes.

Further, according to a desirable embodiment of the present invention, the translucent image generating means further includes display address calculating means for respectively calculating the image display address of the tomographic image data and of the elasticity image data and image generating means for generating a combined image by assigning pixels of the tomographic image data and of the elasticity image data to the calculated display address.

With this structure, the tomographic image and the elasticity image are displayed in a striped pattern or in a check pattern, whereby their positional relation can be easily grasped.


Brief Description of the Drawings

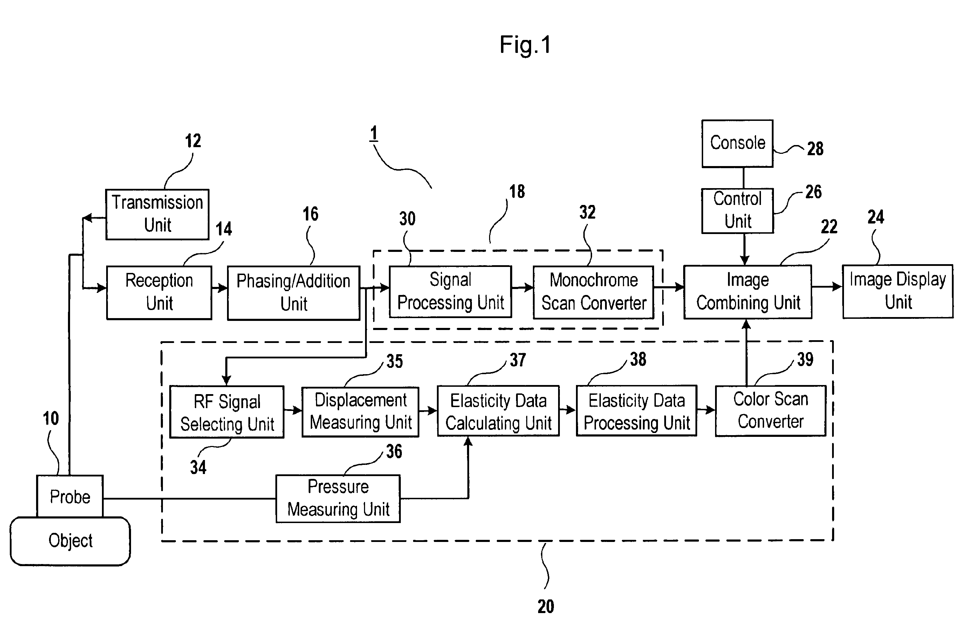

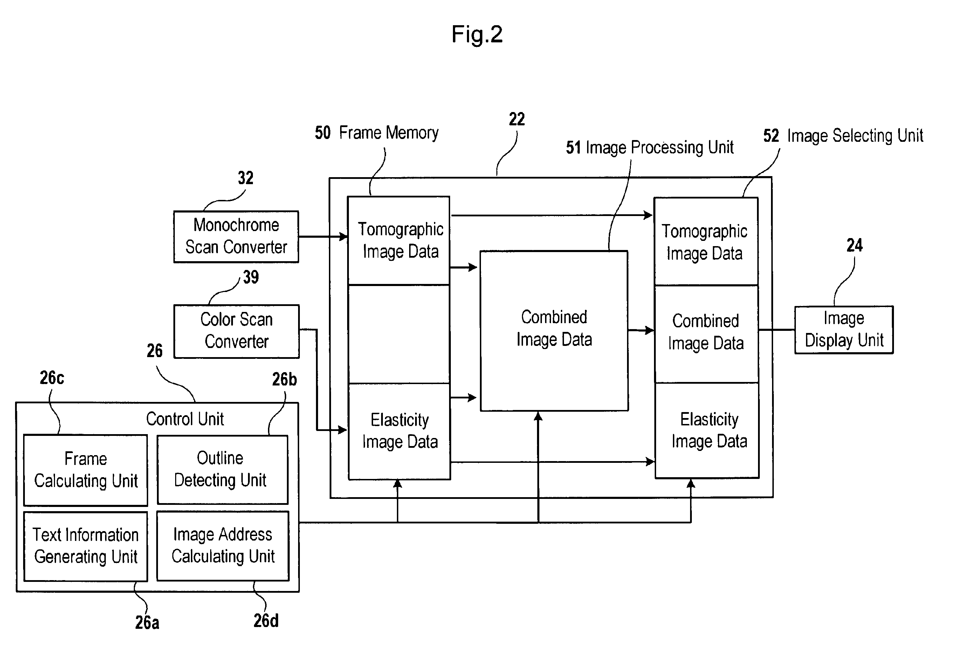

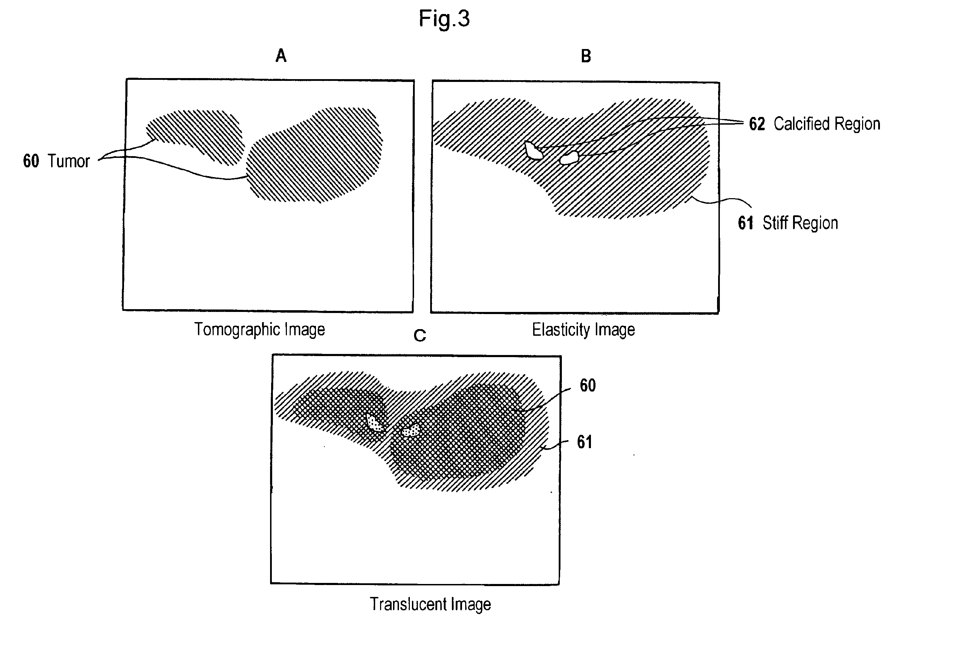

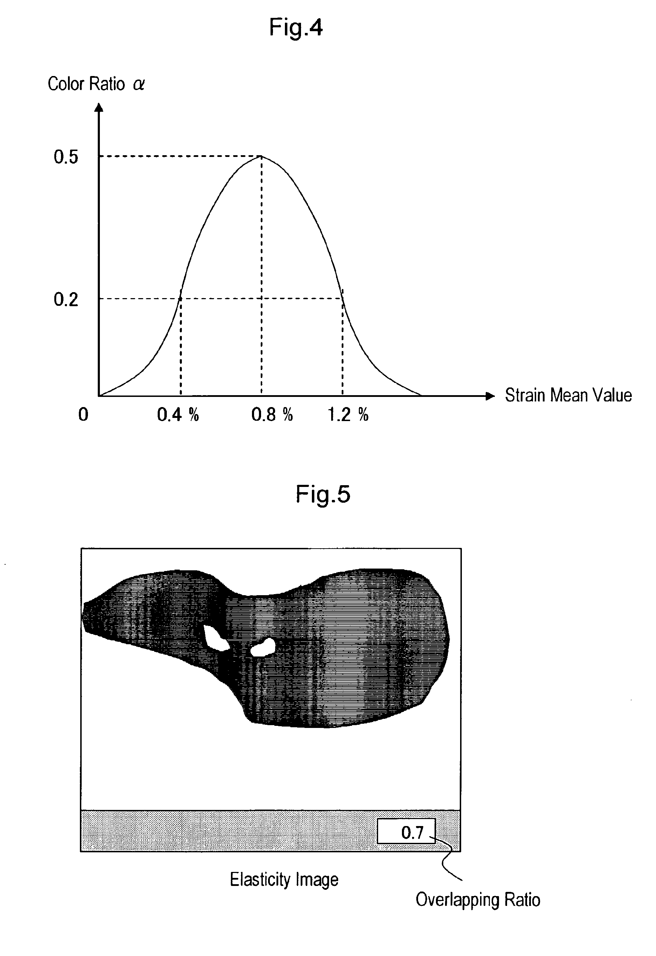

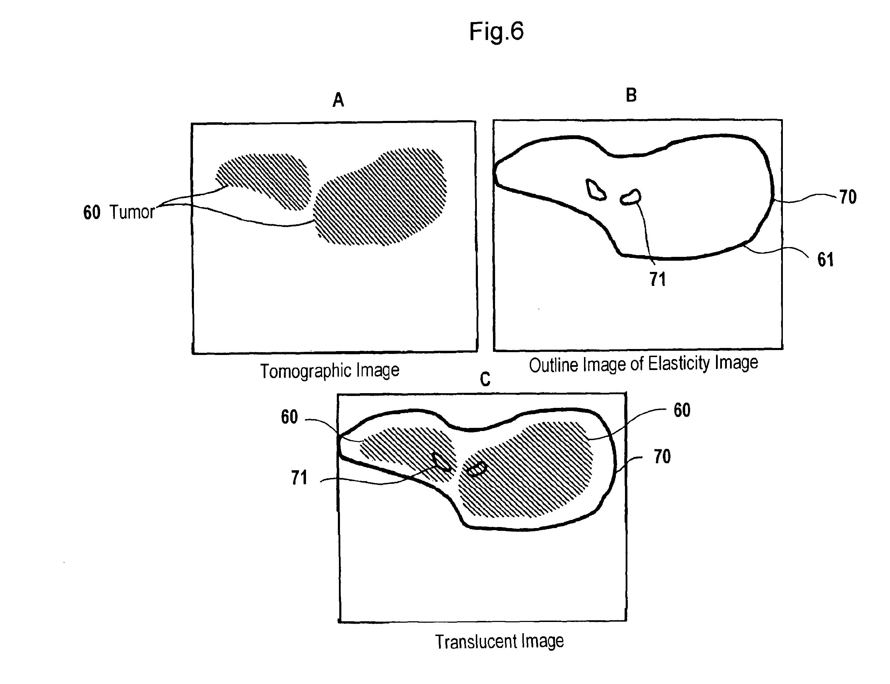

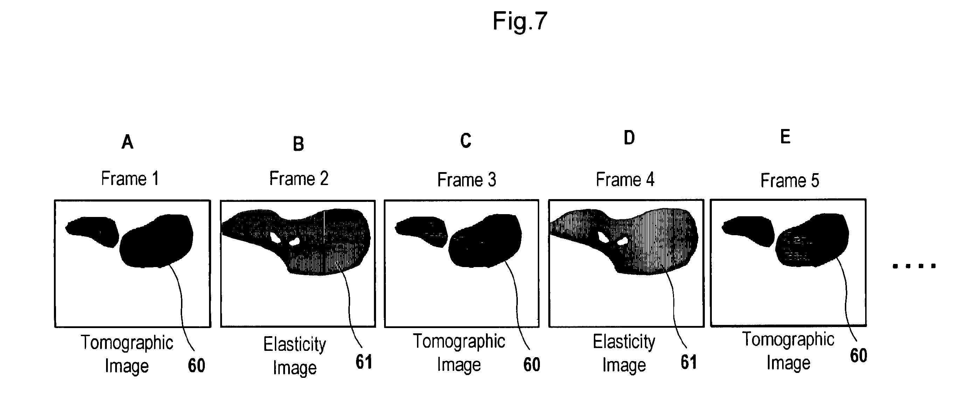

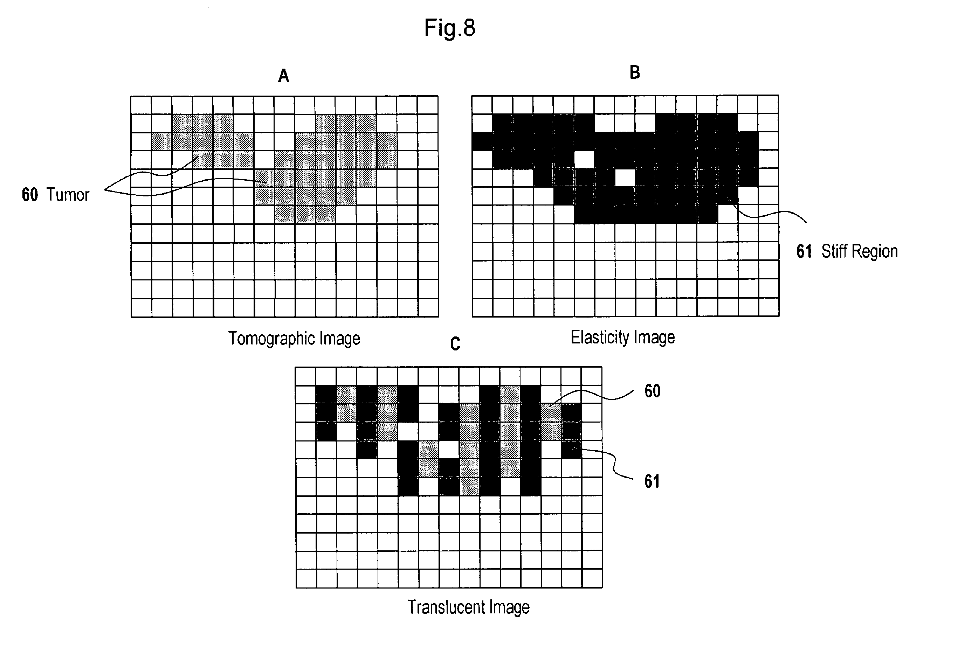

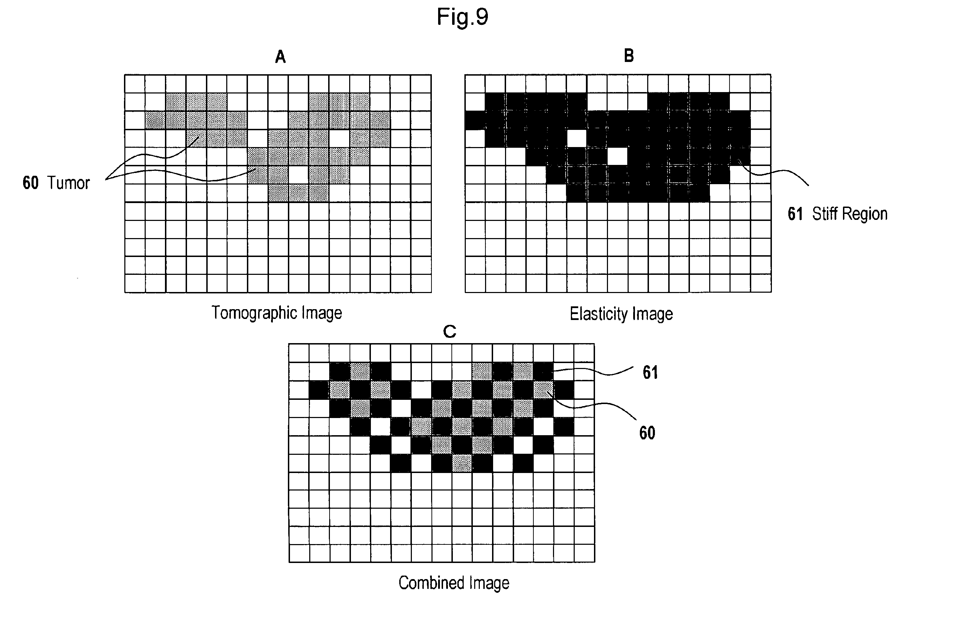

Fig.1 is a block diagram showing a desired embodiment of ultrasound diagnostic apparatus on the basis of the present invention. Fig.2 is a block diagram showing one embodiment of image overlapping unit of Fig.1. Fig.3 is a diagram showing an example of displaying the combined image in which a color elasticity image is turned into a translucent image and superposed on the tomographic image. Fig.4 is a graph showing relation between a mean value of distortion due to pressing speed of the probe to the object and a color ratio in Fig.1. Fig.5 is a diagram showing an example of numerical value display of the combination ratio in the case that the tomographic image and the color elasticity image are displayed as a translucent image. Fig.6 is a diagram showing an example of simulatively displaying a translucent image by displaying an outline extracted from the color elasticity image and the tomographic image according to a desirable embodiment of the present invention. Fig.7 is a diagram showing an example of simulatively displaying the translucent image by switching the tomographic image and the color elasticity image at every frame according to another desirable embodiment of the present invention. Fig.8 is a diagram showing an example of displaying the tomographic image and the color elasticity image on alternate lines according to another desirable embodiment. Fig.9 is a diagram showing an example of displaying the tomographic image and the color elasticity image on alternate pixels according to another desirable embodiment of the present invention.


Best Mode for Carrying Out the Invention

Hereinafter, a desirable embodiment of ultrasound diagnostic apparatus according to the present invention will be described in detail with reference to the accompanying drawings.


(Embodiment 1)

A first embodiment of ultrasound diagnostic apparatus according to the present invention will be described with reference to Figs.1 to 3.

As shown in Fig.1, ultrasound diagnostic apparatus 1 includes probe 10 put onto an object to be examined in use, transmission unit 12 for transmitting an ultrasound wave to the object at time intervals through probe 10, reception unit 14 for receiving a time-sequential reflected echo signal generated from the object, and phasing/addition unit 16 for phasing and adding the received reflected echo and time-sequentially generating an RF signal frame data.

The apparatus also includes tomographic image generating unit 18 for generating a tomographic image, e.

g. a monochrome tomographic image on the basis of the RF signal frame data from phasing/addition unit 16, and elasticity image generating unit 20 for calculating elasticity data by measuring displacement of the object's tissue from the RF signal frame data of phasing/addition unit 16 and generating a color elasticity image. Further, the apparatus also includes image overlapping unit 22 for overlapping the monochrome tomographic image and the color elasticity image and image display unit 24 for displaying the combined image.

Probe 10 is formed by arranging a plurality of transducers and has a function of transmitting and receiving an ultrasound wave to/from the object via the transducers by electrically scanning a beam.

Transmission unit 12 has a function of generating a wave transmission pulse for driving probe 10 to generate an ultrasound wave and of setting a focal point of the transmitted ultrasound wave at a certain depth. Further, reception unit 14 is designed to amplify the reflected echo signal received by probe 10 with a predetermined gain and generate an RF signal, i.e. a received signal.

Tomographic image generating unit 18 has signal processing unit 30 and monochrome scan converter 32. Here, signal processing unit 30 is designed to obtain brightness information and tomographic image data by performing signal processings such as gain correction, logarithmic compression, detection, edge enhancement, filtering processing, and the like on the RF signal frame data input from phasing/addition unit 16. Further, monochrome scan converter 32 has an A/D converter for converting the tomographic image data from signal processing unit 30 into digital signals, a frame memory for time-sequentially storing the converted plural tomographic image data, and a controller. Monochrome converter 32 is designed to acquire as one image the tomographic frame data of inside of the object stored by the controller into the frame memory and convert the acquired tomographic frame data into signals to be read out in synchronism with television operation.

Further, tomographic image generating unit 20 has RF signal selecting unit 34, displacement measuring unit 35, pressure measuring unit 36, elasticity data calculating unit 37, elasticity signal processing unit 38, and color scan converter 39, and is separately provided on the latter part of phasing/addition unit 16.

RF signal selecting unit 34 has a frame memory and a selecting unit. This RF signal selecting unit 34 is designed to store plural RF signal frame data from phasing/addition unit 16 into the frame memory and select one pair, i.e. two sets of RF signal frame data from the stored RF signal frame data group. For example, RF signal selecting unit 34 is designed to sequentially keep into the frame memory the time-sequential RF signal frame data, i.e. the RF signal frame data generated on the basis of the image frame rate, select as first data the presently kept RF signal frame data (N) in response to a command from control unit 26, and simultaneously select one RF signal frame data (X) from among the previously kept RF signal frame data group (N-1, N-2, N-3, ... N-M). Meanwhile, N, M, and X are index numbers assigned to the RF signal frame data, being natural numbers.

Displacement measuring unit 35 is designed to calculate a displacement and the like of the tissue from a pair set of RF signal frame data. For example, displacement measuring unit 35 performs a one-dimensional or two-dimensional correlation processing from the pair set of data, i.e. RF signal frame data (N) and RF signal frame data (X) selected by RF signal selecting unit 34, and calculates a one-dimensional or two-dimensional displacement distribution concerning the displacement in the tissue, a moving vector i.e. the direction of the displacement, and the scale of the displacement corresponding to each point of the tomographic image. Here, detection of the moving vector is performed by means of a block matching method. The block matching method is such that an image is divided into blocks respectively including N x M pixels, a block within an ROI is marked, a block which is most approximated to the marked block is found from the previous frame, and a sample value is determined on the basis of predictive coding, i.e. difference with reference to the approximated block.

Tomographic image generating unit 18 and elasticity image generating unit 20 may use RF signals of an identical timing respectively to obtain a tomographic image and an elasticity image. With this structure, it is possible to share both circuits and improve the frame rate in obtaining the tomographic image and the elasticity image.

Pressure measuring unit 36 is designed to measure and calculate an intracavitary pressure in a diagnostic region of the object. For example, the pressure measuring unit having a pressure sensor is attached to probe 10 put onto the object's body surface, and a stress distribution is given to the body cavity in the object's diagnostic region by pressurizing and depressurizing the head of probe 10. At this time, in an arbitrary time phase, the pressure sensor measures and maintains the pressure applied by the probe head to the body surface.

Elasticity data calculating unit 37 is designed to calculate a distortion and an elasticity ratio of the tissue corresponding to each point of the tomographic image from a measured value, e.g. the moving vector from displacement measuring unit 35 and a pressure value from pressure measuring unit 36, and generate an elasticity image signal, i.e. elasticity frame data on the basis of the distortion and the elasticity ratio.

Here, the data of distortion are calculated by taking a spatial derivative of a distance, e.g. the displacement of the tissue. Further, the data of elasticity ratio are calculated by dividing the pressure displacement by the distance displacement. For example, provided that a displacement measured by displacement measuring unit 35 is $\Delta L$ and a pressure measured by pressure measuring unit 36 is $\Delta P$, the distortion (S) is calculated with a formula $S=\Delta L/\Delta X$ as it can be calculated by taking a spatial derivative of $\Delta L$. Further, a Young's modulus $Y_m$ of the elasticity ratio data is calculated with a formula $Y_m=(\Delta P)/(\Delta L/L)$. Because the elasticity ratio of the tissue corresponding to each point of the tomographic image can be calculated from this Young's modulus $Y_m$, two-dimensional elasticity image data can be sequentially obtained. Meanwhile, a Young's modulus is a ratio of a simple tensile stress applied to an object to a distortion generated in parallel to the tension.

Elasticity data processing unit 38 has a frame memory and an image processing unit, which is designed to keep the elasticity frame data time-sequentially output from elasticity data calculating unit 37 into the frame memory and perform image processings on the kept frame data into the image processing unit in response to a command of control unit 26.

Color scan converter 39 is designed to convert the elasticity frame data from elasticity data processing unit 38 into hue information. That is, it is designed to convert the elasticity frame data into three primary colors, i.e. red (R), green (G), and blue (B). For example, it converts the elasticity data of large distortion into a red code, and converts the elasticity data of small distortion into a blue code.

As shown in Fig.2, image overlapping unit 22 has frame memory 50, image processing unit 51, and image selecting unit 52. Here, frame memory 50 is designed to store the tomographic image data from monochrome scan converter 32 and the elasticity image data from color scan converter 39. Image processing unit 51 is designed to add and combine the tomographic image data and the elasticity image data kept in frame memory 50 at a combination ratio in response to a command of control unit 26. The brightness information and the hue information of each pixel of the combined image are obtained by adding the respective information of the monochrome tomographic image and the color elasticity image at a combination ratio. Further, image selecting unit 52 is designed to select an image to be displayed on image display unit 24 from among the tomographic image data and the elasticity image data in frame memory 50 and the combined image data of image processing unit 51 in response to a command of control unit 26. The ratio setting of combined image data may be performed by selecting at least either the pressure measured by the pressure measuring unit or the elasticity information calculated by the elasticity image generating unit and setting a ratio of overlapping the tomographic image and the elasticity image on the basis of the selected pressure or elasticity information. Further, image overlapping unit 22 may variably set at least either the combination ratio or the ROI in response to a command of control unit 26. By setting the ROI so as to display the elasticity image on a part of the image display area, display time of the elasticity image is shortened, whereby the frame rate of the image combined with the tomographic image can be improved. Further, the image information of the tomographic image may be the brightness information of the tomographic image generated by the tomographic image generating unit. The image information of the elasticity image may be the elasticity information calculated by the elasticity image generating unit. Further, a pressure measuring unit for measuring a pressure of the probe may be provided, and the image information of the elasticity image may be the pressure measured by the pressure measuring unit.

Operations of thus constructed ultrasound diagnostic apparatus 1 will be described. Ultrasound diagnostic apparatus 1 repeatedly transmits ultrasound waves to the object at time intervals via probe 10 put onto the object. Time-sequential reflected echo signals generated from the object are received by reception unit 14 are phased and added to generate RF signal frame data. A tomographic image, e.g. a monochrome B-mode image is obtained by tomographic image generating unit 18 on the basis of the RF signal frame data. At this time, one tomographic image is obtained by scanning an ultrasonic beam in a plurality of directions. Meanwhile, a color elasticity image is obtained by elasticity image generating unit 20 on the basis of the RF signal frame data phased and added by phasing/addition unit 16. A combined image is formed by adding the obtained monochrome tomographic image and color elasticity image in image overlapping unit 22.

Here, one example of processings of image overlapping unit 22 will be described. In the following description, the tomographic image data input into image processing unit 51 is represented as (Tomographic image data)$_{i,j}$ and the elasticity image data are represented as (Elasticity image data)$_{i,j}$. Here, (i,j) represents a coordinate of a data element.

First, the tomographic image data including the monochrome brightness information is converted into hue information. If the tomographic image data after conversion has the same bit length as that of the monochrome brightness information, hue data, i.e. data of three primary colors (RGB) concerning the converted tomographic image data are expressed as following formulas (1):

$$\text{(Tomographic image data R)}_{i,j} = \text{(Tomographic image data)}_{i,j}$$

$$\text{(Tomographic image data G)}_{i,j} = \text{(Tomographic image data)}_{i,j}$$

$$\text{(Tomographic image data B)}_{i,j} = \text{(Tomographic image data)}_{i,j} \tag{1}$$

Next, the converted tomographic image data and the elasticity image data are added and combined at a

**EP 1 554 982 A1**

combination ratio (α). Here, the combination ratio (α) is arbitrarily predetermined by the operator in accordance with characteristics of the tissue, which is a value larger than zero and smaller than 1. With this combination ratio (α), the combined image is generated as shown in formula (2). The combined image is selected by image selecting unit 52 and displayed on display unit 24.

$$(\text{Combined image data R})_{i,j}$$

$$= (1-\alpha) \times (\text{Tomographic image data R})_{i,j} + \alpha \times (\text{Elasticity image data R})_{i,j}$$

$$(\text{Combined image data G})_{i,j}$$

$$= (1-\alpha) \times (\text{Tomographic image data G})_{i,j} + \alpha \times (\text{Elasticity image data G})_{i,j}$$

$$(\text{Combined image data B})_{i,j}$$

$$= (1-\alpha) \times (\text{Tomographic image data B})_{i,j} + \alpha \times (\text{Elasticity image data B})_{i,j} \tag{2}$$

Thus combined image will be described with reference to Figs.3. In Fig.3A, tumor 60 is displayed in a monochrome tomographic image. In Fig.3B, stiff region 61 in the tissue around tumor 60 is displayed as a color elasticity image. In Fig.3C, an image obtained by image overlapping unit 22 overlapping the monochrome tomographic image and the color elasticity image at a desired ratio is displayed.

As shown in Fig.3C, since the combined image is obtained by adding the monochrome tomographic image of Fig.3A and the color elasticity image of Fig.3B in each coordinate at the combination ratio (α), the information of both images is reflected. Accordingly, since in the image shown in Fig.3C a translucent tomographic image of tumor 60 is displayed on the image of stiff region 61, the size and position of tumor 60 can be recognized, whereby a region removed in surgery can be properly determined.

If the superposed both images of the monochrome tomographic image and the color elasticity image can be seen in the translucent display, image overlapping unit 22 may also process the images so that at least either of those images is made translucent in the display.

Further, by changing the combination ratios of the tomographic image and of the elasticity image on the basis of the information of the gain correction and the like of tomographic image, it is possible to display a properly colored image of high diagnosability.

As described above, according to the ultrasound diagnostic apparatus of this embodiment, it is possible to display the elastic behavior of the tissue based on the elasticity image and a distribution of the ultrasound reflection strength, i.e. tissue shape which can be mutually compared on an identical screen, whereby the relation between the tissue and its elastic behavior can be properly diagnosed.

(Embodiment 2)

Next, an example of displaying a numerical value of the combination ratio (α) of the tomographic image and of the elastographic image and variably setting the combination ratio will be described.

Image overlapping unit 22 has text information generating unit 26a included in control unit 26. Text information generating unit 26a, such as a known character generator, is designed, e.g. to display the numerical value of the combination ratio of the monochrome tomographic image data and the elasticity image data when they are superposed in the display.

Here, the reason of displaying the combination ratio and variably setting it with the text information generating unit will be described. As shown in Fig.4, according to a statistical work for a population of a distortion measuring point group within one frame, a distortion image of highest color ratio, i.e. highest contrast is obtained with the distortion amount of, e.g. 0.8% as a distortion mean value. The distortion mean value has characteristics that it correlates with a pressurizing speed, and the mean value becomes higher as the pressing speed is higher and it becomes lower as the pressing speed is lower.

Here, by comparing the distortion mean value with the color ratio, the color ratio becomes higher as the image quality is higher (when the pressurizing speed is optimum). Accordingly, the operator who pressurizes the object with the probe can realize optimum pressurizing movement by observing the elasticity image.

Further, for example, the following procedure is applicable so as to optimize the image quality of the combined image data superposing the tomographic image data and the elasticity image data:

6

(1) Calculate a mean value (Bmean) of brightness of the monochrome tomographic image data.
(2) Calculate a mean value (Emean) of color brightness of the elasticity image data.
(3) Calculate coefficient $\beta$ of Bmean with which a ratio between Bmean and Emean becomes 1:1 (Bmean x $\beta$ = Emean).
(4) Combine the elasticity image data on the tomographic image data at a ratio of $\alpha$ by multiplying each image pixel of the tomographic image data by $\beta$.

Meanwhile, the above combination ratio may also be set in parallel with a gain of the tomographic image data.

After that, the calculated tomographic image data and color elasticity image data are combined by image processing unit 51. As shown in Fig.5, the combined image is selected by image selecting unit 52 to be displayed, and a numerical value of the combination ratio of the elasticity image data is displayed as, e.g. 0.7. Meanwhile, the procedure of combination processings is the same as the procedure described in the first embodiment.

In this manner, the elasticity image including stiff region 61 and the tomographic image including tumor 60 are combined in the display on the basis of the combination ratio. Accordingly, by properly adjusting the combination ratio, mutual relation between the shape of tissue and the elasticity information can be properly diagnosed.

In this case, the combination ratio may be freely set. The setting is arbitrarily changed with a user interface, e.g. console 18. That is, the combination ratios of the tomographic image and of the elasticity image are set in the apparatus in plural steps, e.g. in eight steps, such as $\alpha$ =0.0, 0.2, 0.3, 0.4, 0.5, 0.6, 0.8, 1.0, and any one of the set values can be changed by the user with up/down keys on the console in an arbitrary timing. Further, the combination ratio may also be set at an arbitrary value in the region between 0 and 1, and it may be determined and changed with a rotary key on the console.

Further, when the combination ratios of the tomographic image and of the color elasticity image are set on the basis of the elasticity information calculated by the elasticity image generating unit or the pressure of the probe measured by the pressure measuring unit, the automatically set combination ratio is displayed.

(Embodiment 3)

In this embodiment, an example of superposing an outline, i.e. a borderline of a stiff region of the elasticity image on the tomographic image will be described.

Image overlapping unit 22 has outline detecting unit 26b included in control unit 26 shown in Fig.2. Outline detecting unit 26b is designed to extract a borderline of regions having different elastic behavior in the tissue on the basis of, e.g. a set threshold.

Here, outline detecting unit 26 will be described in detail. In the following description, tomographic image data input to the outline detecting unit is (Tomographic image data X) $_{i,j}$ and image data output from the outline detecting unit is (Outline image data X)$_{i,j}$.

First, outline detecting unit 26b separates (Elasticity image data X)$_{i,j}$ into two values using an image processing filter. For example, a smoothing filter and a median filter are done thereon, and (Image data X)$_{i,j}$ are separated into two values on the basis of threshold (T1) preset by console 28. In this manner, (Elasticity image data X)$_{i,j}$ is distinctly separated into a region smaller than threshold (T1) and a region larger than threshold (T1).

Next, for example, numerical value 1 is substituted for coordinate position (i, j) corresponding to the border between two regions separated in (Elasticity image data X) $_{i,j}$, and numerical value 0 is substituted for other coordinates. Because only the coordinates corresponding to the border of the two regions become numerical value 1, (Outline image data X)$_{i,j}$ displaying only this border are generated.

Moreover, a borderline of other thresholds can also be pictured at the same time. For example, (Elasticity image data X)$_{i,j}$ are separated into two regions on the basis of another threshold (T2) and, e.g. numerical value 2 is substituted for coordinate (i, j) corresponding to the separating border. By extracting the coordinate substituted by numerical value 2 and the coordinate substituted by numerical value 1, the outline image indicating two borderlines is obtainable. In this manner, by setting the thresholds in accordance with the observer's need, an outline image including a plurality of borderlines can be generated.

The obtained outline image and the tomographic image are added and combined by image processing unit 51 on the basis of the combination ratio ($\alpha$). The combined image is selected by image selecting unit 52 and displayed as a translucent image or in another display mode, whereby the positional relation between the tomographic image and the elasticity image can be grasped.

A detailed example of display with this outline detecting processing will be described with reference to Fig.7. Fig.6 is an example of image displayed by performing the processing for overlapping the monochrome tomographic image and the outline image extracted from the elasticity image. As shown in Fig.6A, the displayed monochrome tomographic image includes tumor 60. As shown in Fig.6B, the displayed outline image indicates first borderline 70 detected with the first threshold and second borderline 71 detected with the second threshold. As shown in Fig.6C, a combined image of the monochrome tomographic image and the outline image is displayed. Meanwhile, the procedure of the combination processing is the same as that described in the first embodiment.

As described above, in the image of Fig.6C both borderline 70 of the stiff region including tumor 60 and borderline 71 of, e.g. region 62 in which tissue is calcified are combined with the tomographic image in the display. Accordingly, by observing the image shown in Fig.6C, the mutual relation between the tissue and the borderline indicating elastic behavior difference can be properly diagnosed. In this manner, since shape information obtained in the tomographic image is emphasized in the display, their positional relation becomes more apparent. For example, in the cancer treatment, expanse of stiff region 61 with respect to expanse and size of tumor 60 can be obviously grasped and a removal region of the object can be properly determined.

Further, although an outline image is formed on the basis of the elasticity image in the above example, the outline image may also be made on the basis of the tomographic image. Further, determination of whether the threshold value, the number of threshold, the combination ratio, i.e. overlapping ratio $\alpha$, and the function of outline detecting unit 26b are available or void is arbitrarily changed respectively with the user interface, e.g. console 18.

Further, by displaying the outlines while changing at least one among size, color, and line type in accordance with the size of the displacement and of the pressure, the displacement and the pressure can be identified from the thickness of the outline or the like, whereby it is possible to provide information about the size of the displacement and of the pressure to the operator and the observer.

(Embodiment 4)

Next, an example of simulatively displaying a translucent image by alternately displaying the frame of tomographic image and the frame of elasticity image, and parallelizing the tomographic image and the elasticity image by means of afterimage effect of the observer's eyes will be described.

Image overlapping unit 22 has frame calculating unit 26c included in control unit 26 shown in Fig.2. Frame calculating unit 26c calculates, e.g. a display order of frame memory storing the monochrome tomographic image and the elasticity image.

Frame calculating unit 26c is designed to alternately display the tomographic image including tumor 60 as shown in Figs.7A, 7C, and 7E and the elasticity image including stiff region 61 as shown in Figs.7B and 7D in the order of Figs.7A, 7B, 7C, 7D, 7E.... This alternate display is commanded from console 28 to frame calculating unit 26c so as to alternately display the tomographic images and the elasticity images one by one. Although in this example the tomographic images and the elasticity images are alternately displayed one by one, it is needless to say that the display mode may be freely set, e.g. to display the tomographic image twice and the elasticity image once. Further, in the alternate display of the tomographic image data and the color elasticity image data, the tomographic image and the elasticity image may be obtained by using different frequency to the respective RF signals. By doing so, the tomographic image or the elasticity image of desired resolution is obtainable.

As described above, the elasticity image including stiff region 61 is combined with the tomographic image including tumor 60 by integral effect in the display. Accordingly, when the image alternately showing the tomographic image and the elasticity image is displayed on the image display unit 24 as shown in Fig.7, the tomographic image and the elasticity image can be parallelized by means of the afterimage effect of the observer's eyes. In this manner, e.g. in the cancer treatment, the expanse of stiff region 61 with respect to the expanse and the size of tumor 60 can be mutually compared, whereby the relative difference in their expanse is apparently grasped and the removal region of the object can be properly determined.

In this case, the mode of alternate image display may be freely set. The setting is arbitrarily changed with the user interface, e.g. console 18.

(Embodiment 5)

In this embodiment, an example of displaying the elasticity image and the tomographic image alternately in predetermined pixel rows (lines) or pixels will be described.

Image overlapping unit 22 has image address calculating unit 26d included in control unit 26 shown in Fig.2. Image address calculating unit 26d is designed to calculate a display address of the monochrome tomographic image data and the elasticity image data.

Image address calculating unit 26d is designed to calculate rows to display the tomographic image data including tumor 60 as shown in Fig.8A and the elasticity image data including stiff region 61 as shown in Fig.8B. Here, calculation is performed as shown in Fig.8, provided that an odd row shows the tomographic image data and an even row shows the elasticity image data. It is needless to say that this calculation is a mere example, and various modes of displaying those image data on alternate pixel rows (lines) by, e.g. counterchanging the odd and even rows, or by applying the calculation to lines (depth direction) instead of rows, and the like, may be included.

Further, image address calculating unit 26d is designed to calculate the pixels to display the tomographic image data as shown in Fig.9A and the elasticity image data as shown in Fig.9B. Here, it is designed to calculate pixels alternately displaying the tomographic image data and the elasticity image data.

Further, the translucent image data are simulatively generated from the calculated tomographic image data and the calculated color elasticity image data on the basis of desired α.

As described above, in the images of Figs.8C and 9C the elasticity image data including stiff region 61 and the tomographic image data including tumor 60 are alternately displayed in each pixel row (line) or in each pixel. Accordingly, by observing the images shown in Figs.8C and 9C where the tomographic image and the elasticity image are displayed in a striped pattern or in a check pattern, it becomes easy to grasp their positional relation. Therefore, for example, in the cancer treatment the expanse of stiff region 61 can be compared with the expanse and the size of tumor 60, whereby the relative difference in their expanse can be apparently grasped and the removal region of the object can be properly determined.

Although rows, lines, or pixels are alternately displayed in this example, the alternate rows, lines, and pixels may be freely set. The setting is arbitrarily changed with the user interface, e.g. console 18.

Although five desirable embodiments have been described above, the ultrasound diagnostic apparatus according to the present invention is not limited thereto. For example, the combination processing can be performed by image processing unit 22 on a predetermined particular display region (ROI). That is, when elasticity image generating unit 20 generats a color elasticity image while adding hue to areas indicating different elastic behavior in the object's tissue, it is possible to limit the region of image generation to a predetermined ROI in response to a command of control unit 26 in generating an image. Accordingly, time taken for image generation can be shortened in comparison with the case of generating the color elasticity image of the whole tissue of the object, whereby the frame rate of display image can be improved. Meanwhile, the ROI, i.e. a set region on the display image can be arbitrarily changed with the user interface, e.g. console 28. Further, each of those embodiments may be set as an individual display mode and selected by image selecting unit 52, and a method of switching those display mode is also applicable.

Further, although image selecting unit 52 according to the above embodiments selects the combined image of the monochrome tomographic image and the elasticity image to be displayed on display unit 24, it is also possible to arbitrarily select the image to be displayed. For example, while a tomographic image, an elasticity image, a combined image, an outline image of tomographic image, an outline image of elasticity image, and the like are generated, image selecting unit 52 can select a plurality of arbitrary images among those generated images and display them on the same screen. By doing so, for example, it is possible to select the tomographic image and the combined image and arregion both of them in the display, whereby it is possible to recognize the mutual relation of the form and the elastic behavior of the tissue on the combined image, and, after that, recheck the relation by comparing the combined image with a tomographic image displayed on the same screen.

Further, although displacement measuring unit 35 according to the above embodiments uses the block matching method in detecting a moving vector of the tissue corresponding to each point of the tomographic image, a technique of a gradient method or the like may be used instead. For example, it is possible to use a method of calculating the displacement by calculating autocorrelation of data on an identical region of two images.

Further, although probe 10 according to the above embodiments is the electric scan type formed by plurality of arregiond transducers, it may be the mechanical scan type formed by one transducer. Further, while this probe 10 is put onto the object's body surface, the embodiments are also applicable to a transesophageal probe and an intravascular probe.

Further, although in the image combination processing according to the above embodiments the monochrome tomographic image and the color elasticity image are combined, the combination processing may be changed in accordance with characteristics of the tissue. For example, a tomographic image provided with blue hue and a shading elasticity image provided with red hue may be combined.

Further, although elasticity data calculating unit 37 according to the above embodiments generates the elasticity image data by calculating the distortion of tissue (S) and the Young's modulus $Y_m$, the elasticity ratio may be generated instead by calculating parameters indicating stiffness and characteristics of arterial wall, such as a stiffness parameter (β), pressure-elasticity coefficient (Ep), increment-elasticity coefficient (Einc), and the like.

According to those embodiments, since the brightness information and the hue information of each pixel of the combined image are obtained by adding the pixel information of the tomographic image and of the color elasticity image at a combination ratio, the combination image includes information of both images. Accordingly, in the combined image, not only the border between different tissues and movement and shape displacement of the tissue are displayed, but also the elasticity information of each region of those tissue are displayed as a color translucent image. Therefore, the relation between the tissue and its elastic behavior is properly diagnosable.

As a result, for example, when the tumor exists in the tissue, a stiff region exists around the tissue and an image reflecting both the size of the tumor and the expanse of the stiff region is obtained. Therefore, it is possible to relatively compare the expanse of the stiff region and the size of the tumor and properly determine the removal region of cancer or the like.

In this case, by weighting the combination ratio, i.e. weighting at least either the tomographic image data or the color elasticity image, it is possible to obtain a desired translucent image which emphasizes either image of tissue shape based on the tomographic image or the color elasticity image.

Further, when a combined image is formed, it is desirable that the image generating unit converts the brightness information of the tomographic image into light's three primary colors, i.e. RGB signals and adds the converted signals to the hue information of the color elasticity image at the combination ratio.

Further, it is desirable to form a color elasticity image of a predetermined ROI. By doing so, the elasticity image generating unit can generate a color elasticity image of a particular tissue within the ROI of the object. Accordingly, in comparison with the case of generating a color elasticity image of the whole tissue, time for generating the color image can be shortened and the frame rate of the display image can be improved.

Further, the combination ratio and the ROI may be variably set, whereby it is possible to arbitrarily set the respective parameters including the combination ratio and the ROI in accordance with characteristics of the object's tissue observed.

Further, instead of adding and overlapping the tomographic image and the color elasticity image at the combination ratio, it is also desirable to provide an outline detecting unit for detecting a borderline between tissues having different elastic behavior in the color elasticity image on the basis of a set threshold of the elasticity information and superpose thus-detected borderline on the tomographic image in the display.

By doing so, not only the movement and the shape displacement of the tissue are displayed, but also the borderline, i.e. the outline between the tissues having different elastic behavior is superposed in the display at the same time. Accordingly, the mutual relation between the movement and the shape displacement of the tissue and its elastic behavior can be properly diagnosed.

An image selecting unit for selecting at least two among four images of the above-formed tomographic image, color elasticity image, combined image thereof, and borderline image is provided, whereby the selected two images can be arregiond on two display area on the same screen of the display unit.

By doing so, for example, if the combined image and the tomographic image are selected and displayed, it is possible to perceive the mutual relation between the shape and the elastic behavior of the tissue on the combined image, and, after that, recheck this relation by comparing the combined image with the tomographic image displayed on the screen.

Further, while those embodiments are described for a method of performing data processings on the image data including the tomographic image data and the elasticity image data, the images may also be combined in the display after changing the display memory of the tomographic image and of the elasticity image to adjust the display position thereof.

**Claims**

1. An ultrasound diagnostic apparatus comprising:

   a probe which is put onto an object to be examined for transmitting and receiving an ultrasound wave;
   means for generating a tomographic image by receiving a reflected echo signal corresponding to the transmitted ultrasound wave;
   means for measuring a displacement of the object's tissue and calculating elasticity information from a reflected echo signal corresponding to an ultrasound wave while a pressure to be applied is changed when the probe is put on the object, and generating a color elasticity image from the elasticity information;
   means for generating a translucent image on the basis of image information of at least one of the tomographic image and the color elasticity image; and
   means for selectively displaying the tomographic image, the color elasticity image, and the translucent image.

2. An ultrasound diagnostic apparatus according to claim 1, wherein the translucent image generating means further includes means for overlapping the tomographic image generated by the tomographic image generating means and the color elasticity image generated by the elasticity image generating means to generate a translucent image on the basis of a desired overlapping ratio.

3. An ultrasound diagnostic apparatus according to claim 2, wherein the translucent image generating means generates a translucent image by determining the overlapping ratio between the tomographic image and of the color elasticity image on the basis of the elasticity information calculated by the elasticity image generating means.

4. An ultrasound diagnostic apparatus according to claim 2, wherein the translucent image generating means further includes pressure measuring unit attached to the probe for measuring information of the pressure applied to the object, and generates translucent image data by setting a desired combination ratio of the color elasticity image data calculated by the elasticity image generating means and of the tomographic image data calculated by the tomographic image generating means on the basis of the pressure information measured by the pressure measuring unit.

5. An ultrasound diagnostic apparatus according to claim 2, wherein the translucent image generating means further includes means for calculating a mean value of the tomographic image, means for calculating a mean value of color brightness of the elasticity image, means for calculating a coefficient with which those calculated mean values are equalized, and means for calculating a combination ratio of the elasticity image by multiplying each image pixel of the tomographic image by the calculated coefficient.

6. An ultrasound diagnostic apparatus according to claim 4, wherein the translucent image generating means further includes means for variably setting a region of interest (ROI) of the color elasticity image data, and forms translucent combined image data of the ROI.

7. An ultrasound diagnostic apparatus according to claim 2, wherein the translucent image generating means includes calculating means for separating the brightness information of the tomographic image into three primary colors and calculating translucent image information from the separated three primary colors and hue information of the color elasticity image.

8. An ultrasound diagnostic apparatus according to claim 1, wherein the display means further includes means for selectively displaying a numerical value of either combination ratio of the elasticity image or that of the tomographic image.

9. An ultrasound diagnostic apparatus according to claim 1, wherein the translucent image generating means further includes an outline detecting means for detecting a borderline between tissues having different elastic behavior in the color elasticity image on the basis of the set threshold of the elasticity information calculated by the elasticity image generating unit and means for generating a combined image of the detected outline and the generated tomographic image.

10. An ultrasound diagnostic apparatus according to claim 8, wherein the outline detecting means changes at least one among thickness, color, and line type of the borderline on the basis of the elasticity information calculated by the elasticity image generating means.

11. An ultrasound diagnostic apparatus according to claim 1, wherein the display selecting means further includes means for respectively storing the tomographic image information, the elasticity image information, and the translucent combined image information, and switches the stored tomographic image information and elasticity image information to selectively display them on the display unit.

12. An ultrasound diagnostic apparatus according to claim 11, wherein the storing means further includes means for storing the tomographic image data, the elasticity image data, and the translucent combined image data and arbitrarily setting a timing of switching those image data.

13. An ultrasound diagnostic apparatus according to claim 1, wherein the translucent image generating means further includes display address calculating means for respectively calculating an image display address of the tomographic image data and of the elasticity image data, and image generating means for assigning pixels of the tomographic image data and the elasticity image data to the calculated display address and generating the combined image.

14. An ultrasound diagnostic apparatus according to claim 13, wherein the display address calculating means performs calculation so as to display the tomographic image data and the color elasticity image data in a striped pattern.

15. An ultrasound diagnostic apparatus according to claim 13, wherein the display address calculating means performs calculation so as to display the tomographic image data and the color elasticity image data in a check pattern.

16. An ultrasound diagnostic apparatus according to claim 1, wherein the image information of the tomographic image is gain information of the tomographic image generated by the tomographic image generating means.

17. An ultrasound diagnostic apparatus according to claim 1, wherein the image information of the color elasticity image is the elasticity information calculated by the elasticity image generating unit.

18. An ultrasound diagnostic apparatus according to claim 1, wherein the translucent image generating means further includes means for forming a translucent combined image of the tomographic image generated by the tomographic image generating means and the color elasticity image generated by the elasticity image generating means on the basis of a desired combination ratio.

19. An ultrasound diagnostic apparatus according to claim 2, wherein the translucent image generating means forms a translucent combined image while setting combination ratios of the tomographic image and of the color elasticity image on the basis of the elasticity information calculated by the elasticity image generating unit.

20. An ultrasound diagnostic apparatus according to claim 2, wherein the translucent image generating means further includes pressure measuring means attached to the probe for measuring information of the pressure applied by the probe to the object, and obtains a translucent image data while setting a desired combination ratio of the tomographic image data and of the color elasticity image data on the basis of the pressure information measured by the pressure measuring unit.

Fig.1

# Fig.2

# Fig.3

A

B

**60** Tumor

**62** Calcified Region

**61** Stiff Region

Tomographic Image

Elasticity Image

C

**60**

**61**

Translucent Image

EP 1 554 982 A1

# Fig.4

Color Ratio $\alpha$

0.5

0.2

0    0.4 %    0.8 %    1.2 %    Strain Mean Value

# Fig.5

0.7

Elasticity Image

Overlapping Ratio

# Fig.6

### A

60 Tumor

Tomographic Image

### B

70

61

71

Outline Image of Elasticity Image

### C

60

71

60

70

Translucent Image

EP 1 554 982 A1

Fig.7

A

Frame 1

Tomographic
Image

**60**

B

Frame 2

Elasticity
Image

**61**

C

Frame 3

Tomographic
Image

**60**

D

Frame 4

Elasticity
Image

**61**

E

Frame 5

Tomographic
Image

**60**

# Fig.8

A

**60** Tumor

Tomographic Image

B

**61** Stiff Region

Elasticity Image

C

60

61

Translucent Image

EP 1 554 982 A1

## Fig.9

A

**60** Tumor

Tomographic Image

B

**61** Stiff Region

Elasticity Image

C

**61**

**60**

Combined Image

EP 1 554 982 A1

**EP 1 554 982 A1**

<table>
<tr><td colspan="2" align="center">**INTERNATIONAL SEARCH REPORT**</td><td>International application No.<br>PCT/JP03/13301</td></tr>
</table>

A. CLASSIFICATION OF SUBJECT MATTER
    Int.Cl⁷ A61B8/08

According to International Patent Classification (IPC) or to both national classification and IPC

B. FIELDS SEARCHED

Minimum documentation searched (classification system followed by classification symbols)
    Int.Cl⁷ A61B8/00-8/15

Documentation searched other than minimum documentation to the extent that such documents are included in the fields searched
    Jitsuyo Shinan Koho          1922-1996    Jitsuyo Shinan Toroku Koho    1996-2003
    Kokai Jitsuyo Shinan Koho    1971-2003    Toroku Jitsuyo Shinan Koho    1994-2003

Electronic data base consulted during the international search (name of data base and, where practicable, search terms used)
    JMEDPlus FILE(JOIS)

C. DOCUMENTS CONSIDERED TO BE RELEVANT

| Category* | Citation of document, with indication, where appropriate, of the relevant passages | Relevant to claim No. |
|---|---|---|
| X<br>Y<br><br>A | JP 5-317313 A (Ken ISHIHARA, Hitachi Medical Corp.),<br>03 December, 1993 (03.12.93),<br>Full text; all drawings<br>(Family: none) | 1<br>2-4,6,7,<br>17-20<br>5 |
| Y<br><br>A | JP 6-178777 A (Aloka Co., Ltd.),<br>28 June, 1994 (28.06.94),<br>Full text; all drawings<br>(Family: none) | 2-4,6,7,<br>17-20<br>1,5 |
| A | Naotaka NITTA et al., "Cho'onpa ni yoru Soshiki no Hisenkei Dansei Tokusei no Gazoka", The Transactions of the Institute of Electronics, Information and Communication Engineers A, 01 December, 2001 (01.12.01), No.12, pages 1405 to 1413 | 1-7,17-20 |

☒ Further documents are listed in the continuation of Box C.   ☐ See patent family annex.

| | |
|---|---|
| *       Special categories of cited documents:<br>"A"   document defining the general state of the art which is not considered to be of particular relevance<br>"E"   earlier document but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search<br>    23 February, 2004 (23.02.04) | Date of mailing of the international search report<br>    09 March, 2004 (09.03.04) |
|---|---|
| Name and mailing address of the ISA/<br>    Japanese Patent Office | Authorized officer |
| Facsimile No. | Telephone No. |

Form PCT/ISA/210 (second sheet) (July 1998)

**EP 1 554 982 A1**

C (Continuation). DOCUMENTS CONSIDERED TO BE RELEVANT

| Category* | Citation of document, with indication, where appropriate, of the relevant passages | Relevant to claim No. |
|---|---|---|
| A | JP 10-105678 A (Toshiba Corp.), 24 April, 1998 (24.04.98), Full text; all drawings & US 2001/0024516 A1 | 1-7,17-20 |

Form PCT/ISA/210 (continuation of second sheet) (July 1998)

# EP 1 554 982 A1

## INTERNATIONAL SEARCH REPORT

International application No.

PCT/JP03/13301

**Box I   Observations where certain claims were found unsearchable (Continuation of item 2 of first sheet)**

This international search report has not been established in respect of certain claims under Article 17(2)(a) for the following reasons:

1. ☐ Claims Nos.:

   because they relate to subject matter not required to be searched by this Authority, namely:

2. ☐ Claims Nos.:

   because they relate to parts of the international application that do not comply with the prescribed requirements to such an extent that no meaningful international search can be carried out, specifically:

3. ☐ Claims Nos.:

   because they are dependent claims and are not drafted in accordance with the second and third sentences of Rule 6.4(a).

**Box II   Observations where unity of invention is lacking (Continuation of item 3 of first sheet)**

This International Searching Authority found multiple inventions in this international application, as follows:

    The technical feature common to the inventions of claims 1-20 is a technical
    feature disclosed in claim 1.  However, the search has revealed that the
    technical feature of claim 1 is not novel since it is disclosed in document
    JP 5-317313 A (Ken ISHIHARA), 03 December, 1993 (03.12.93).
    Accordingly, the technical feature of claim 1 cannot be a special technical
    feature within the meaning of PCT Rule 13.2, second sentence.
    Consequently, it is obvious that the inventions of claims 1-7, 17-20, the
    inventions of claims 8, 10, the invention of claim 9, the inventions of claims
    11, 12, the inventions of claims 13-15, and the invention of claim 16 do not
    satisfy the requirement of unity of invention.

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:  1-7, 17-20

**Remark on Protest**    ☐    The additional search fees were accompanied by the applicant's protest.

   ☐    No protest accompanied the payment of additional search fees.

Form PCT/ISA/210 (continuation of first sheet (1)) (July 1998)

23